# EUROPEAN PATENT SPECIFICATION

(11) **EP 1 035 120 B1**
(45) Date of publication and mention of the grant of the patent: **30.07.2003**
(21) Application number: 99301855.5
(22) Date of filing: 10.03.1999
(51) Int. Cl.: C07D 311/72, A61K 31/335

(54) **Alpha-tocopherol cyclopropylates, the new vitamin E derivatives, and method for producing the same**
Cyclopropylestern von Alfa-Tocopherol, Vitamin E Derivate und Verfahren zu deren Herstellung
Cyclopropylates d'alfa-tocophérol, les dérivés de vitamine E, et méthodes pour les préparer

(43) Date of publication of application: 13.09.2000
(73) Proprietor: SK Corporation, Yongdungpo-ku, Seoul 150-010 (KR)
(72) Inventor: Lee, Si Joon, Yusung-ku, Taejon 305-390 (KR); Cheong, Heui Young, Yusung-ku, Taejon 305-390 (KR)
(74) Representative: Wilkinson, Stephen John

(56) References cited:
- FR-A- 2 278 334

## Description

The present invention relates, in general, to novel alpha-tocopherol cyclopropylates, new vitamin E derivatives and to a method for producing the same and, more particularly, to saturated cyclohydrocarbon carboxy esters of fat-soluble vitamin E, i.e., novel alpha-tocopherol cyclopropylates represented by the following formula I, which comprise cyclohydrocarbon carboxylic acid with a cyclopropyl functional group, and to a method for producing the same: 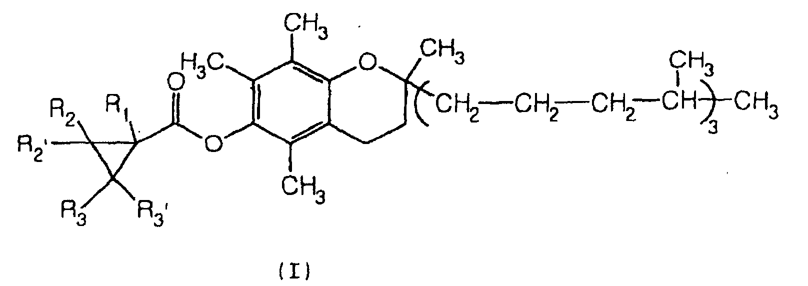 wherein R₁, R₂, R_{2'}, R₃ and R_{3'}, which may be the same or different, each is a hydrogen atom, a linear or branched C₁-C₁₀ alkyl chain, phenyl, acetyl or halogen.

### BACKGROUND OF THE INVENTION

Cyclopropane carboxylic acid derivatives are generally represented by the following formula II: 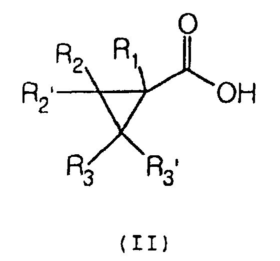 wherein R₁, R₂, R_{2'}, R₃ and R_{3'}, which may be the same or different, each is a hydrogen atom, a linear or branched C₁-C₁₀ alkyl chain, phenyl, acetyl or halogen.

The compounds represented by the formula II have been widely used as a functional group having a specific activity in the compounds recently used in various kinds of medicines and agricultural medicines. Various kinds of derivatives, such as cyclopropane carboxylic acids and ester derivatives thereof, cyclopropane methanol have been widely used in commercialized pyrenoids insecticides and antibiotics, and are very important in medicine/agricultural medicine fields in various forms (see US 5 633 410).

In addition, vitamin E (DL-alpha-tocopherol), represented by the following formula III, is a physiologically essential vitamin among the various vitamins, like vitamin A, C, D, K, beta-carotene and so forth. Vitamin E is relatively complicated in its structure and is widely used as a nutrient, medicines, parturifacient, antioxidant and so forth:

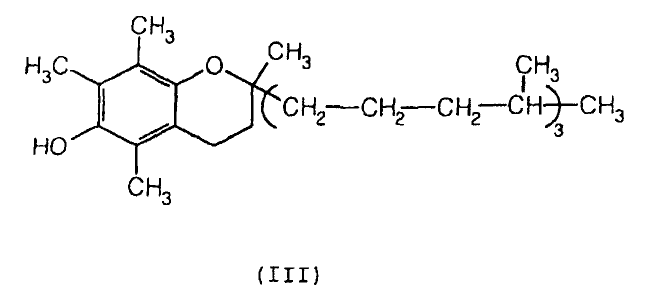

In spite of its importance, Vitamin E, a compound possessing a benzene ring to which hydroxy group binds, is unstable because it itself is easily oxidized. Tocopherol quinone, an oxidized product of vitamin E, no longer has the biological activity of vitamin E. Thus, because storage and management of vitamin E in itself are difficult, there are many attempts in order to increase stability of vitamin E by converting it to other derivative forms.

Representatives of the above mentioned derivatives are esters, which have an enhanced stability by converting the hydroxyl group in phenol ring, specially weak to oxidize, to an ester form. In the above manner, various tocopherols have been made, and examples of such derivatives include tocopherol acetate (US 5 468 883), tocopherol succinate (US 3 538 119), tocopherol phosphate Na⁺ salt (JP 100457823) and, so on on, which are represented by the following formula IV:

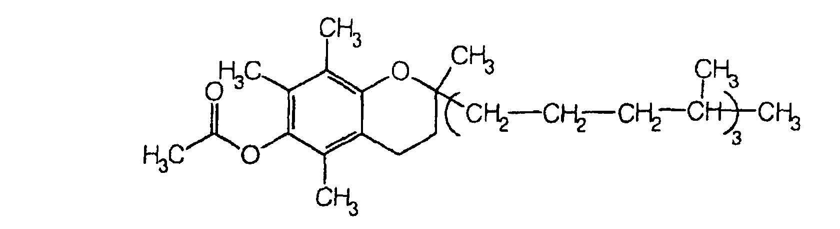

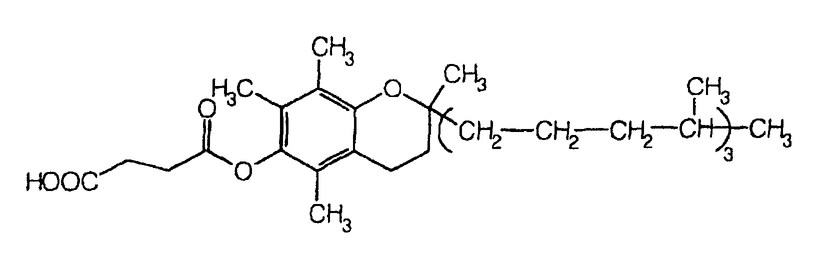

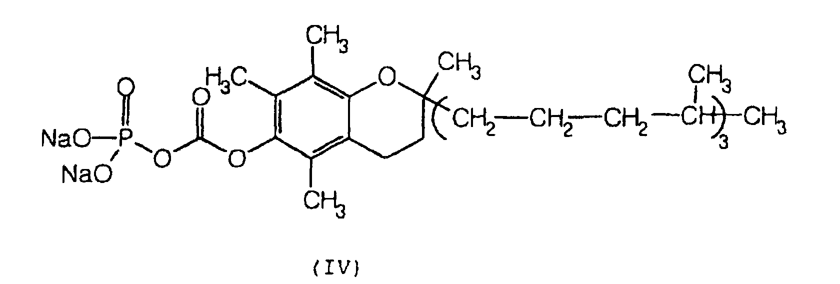

Because these esters have pharmaceutically acceptable stability in addition to being very easy to handle, they are effectively used as replacements for vitamin E.

Upon oral administration, the vitamin E esters are absorbed into the intestinal track and hydrolyzed completely, that is, into free tocopherol and acids by pancreatic enzymes and intestinal enzymes. Thus, the vitamin E esters are absorbed in the form of free tocopherol into the living body. In other words, the esters, when used as replacements for vitamin E in the living body, are converted into free vitamin E and absorbed in the living body, so that the biological effect they have on the living body is practically equivalent to that of vitamin E.

Vitamin E derivatives represented by the following formula V has been filed by the present applicant on April 28, 1997 in Korea under Patent Application No. 97-15961: 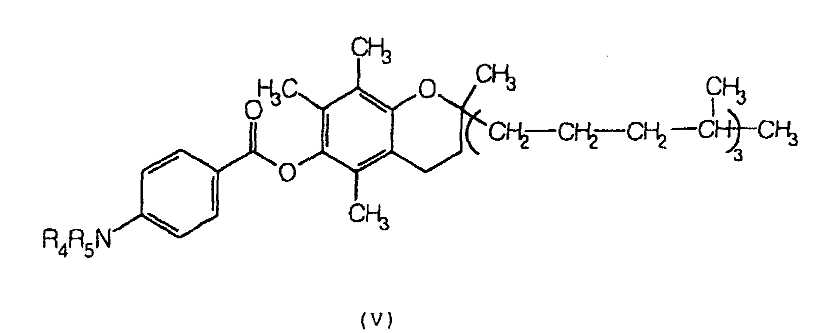 wherein R₄ and R₅ which may be the same of different, each is a hydrogen atom or a linear or branched C₁-C₄ alkyl chain.

### DETAILED DESCRIPTION

Therefore, it is an object of the present invention to provide novel pharmaceutically active compounds which have the equal biological activity to that of vitamin E but a more beneficial effect on the living body.

It is another object of the present invention to provide a method for producing such a compound.

In accordance with an embodiment of the present invention, there is provided a compound represented by the following formula I: 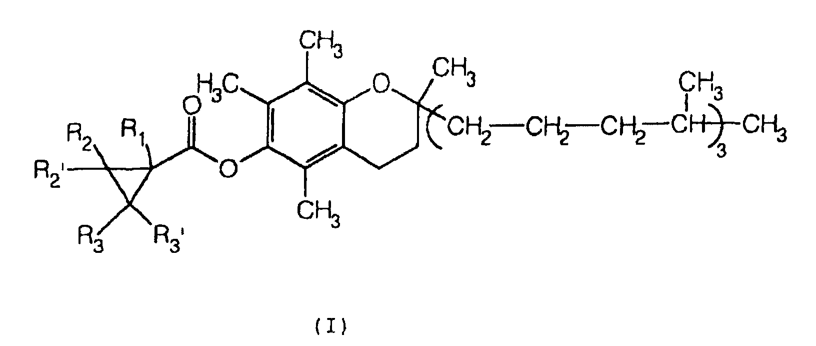 wherein R₁, R₂, R_{2'}, R₃ and R_{3'}, which may be the same or different, each is a hydrogen atom, a linear or branched C₁-C₁₀ alkyl chain, phenyl, acetyl or halogen.

In accordance with another embodiment of the present invention, there is provided a method for producing the compound of the formula I, comprising the reacting cyclopropane carboxylic acid halide produced by using the cyclopropane carboxylic acid represented by the following formula II as starting material: 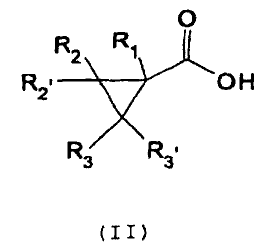 wherein R₁, R₂, R_{2'}, R₃ and R_{3'} are as defined above, with alpha-tocopherol represented by the following formula III: 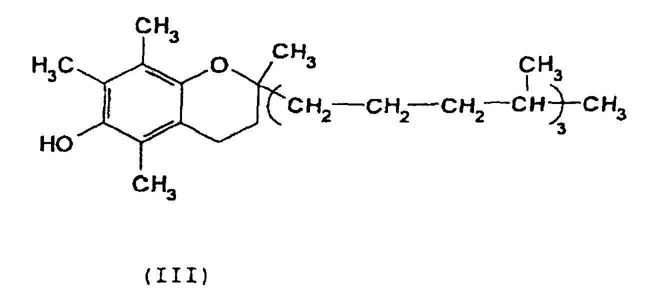 in a solvent and in the presence of a base catalyst.

### BRIEF DESCRIPTION OF DRAWINGS

Figure 1 shows an infrared adsorption spectrum spectroscopy of the compound prepared according to Example I.
Figure 2 shows an ¹H-NMR spectrum of the compound prepared according to Example I.
Figure 3 shows a ¹⁴C-NMR spectrum of the compound prepared according to Example I.
Figure 4 shows a mass spectrum of the compound prepared according to Example I.

### BEST MODE FOR CARRYING OUT THE INVENTION

The compounds of the formula I, consisting of cyclopropane carboxylic acid and alpha-tocopherol, are a kind of representative esters but, to our knowledge, novel compounds which have not been reported, thus far.

These novel compounds may be prepared by the esterification of alpha-tocopherol with cyclopropane carboxylic acid halide. A useful cyclopropane carboxylic acid halide may result from the reaction of thionyl chloride with cyclopropane carboxylic acid, as shown in the following reaction scheme I: 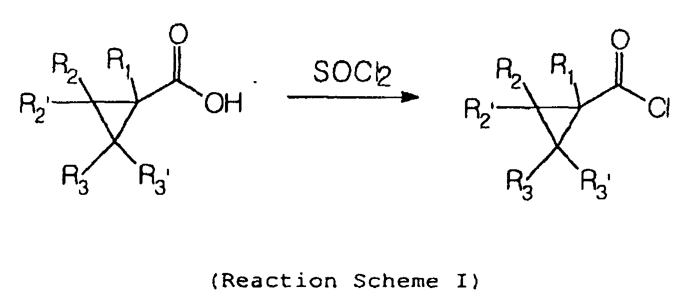 wherein R₁, R₂, R₂, R₃ and R_{3'}, which may be the same or different, each is a hydrogen atom, a linear or branched C₁-C₁₀ alkyl chain, phenyl, acetyl or halogen.

The cyclopropane carboxylic acid halide was prepared by using a modification of procedure described in a prior literature. Without using a particular solvent, the reaction was carried out: thionyl chloride was used as a reactant as well as a solvent. After completion of the reaction, un-reacted thionyl chloride and hydrochloric acid, a by-product, were removed by heating and fractional distillation depending upon the kind of cyclopropane carboxylic halides which were prepared, and the residue was distilled in vacuo to obtain cyclopropane carboxylic acid halide.

The preparation of the alpha-tocopherol cyclopropylate represented by the formula I is shown in the following reaction scheme II: 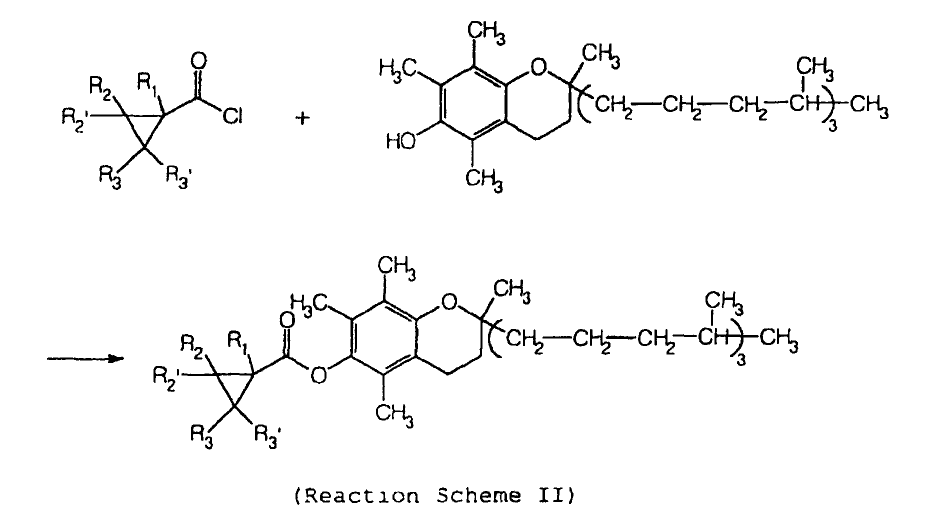 wherein R₁, R₂, R_{2'}, R₃ and R_{3'} are as defined above.

This reaction can be effectively carried out in the presence of a base such as tertiary amine in an aprotic solvent or an aromatic hydrocarbon solvent.

Aprotic solvents are selected from the group consisting of acetonitrile, tetrahydrofuran, dichloromethane and so forth. Aromatic hydrocarbon solvents are selected from the group consisting of benzene, toluene and so on, but acetonitrile is preferable in view of reaction rate and yield.

In addition, as for the base, it may be selected from the group consisting of pyridine, pyridine/4-(dimethylamino)pyridine and triethylamine. While the first two show similar reaction rate and yield each other, triethylamine is relatively poorer in reaction rate and yield.

Generally, the ratio of the reactants and the base, for example, the molar ratio of alpha-tocopherol:cyclopropane carboxylic acid halide:base, is in a range of 1.0:1.5-8.0:1.2-5.0 and preferably 1.0:4.0-7.0:1.5-2.5.

This reaction is carried out at a temperature range from 0 °C to the boiling point of the solvent (about 120 °C), and preferably from room temperature to 35 °C.

The reaction came to the end at 5 hours after the initiation, and preferably the reaction is carried out for about 4-5 hours.

The objective product may be isolated and purified through solvent extraction from the reaction mixture, tube chromatography and/or recrystallization. Where undissolved products are present in the reaction mixture, they are first removed by filtration, and followed by the purification for the final product through the same techniques.

A better understanding of the present invention may be obtained in light of the following examples which are set forth to illustrate, but are not to be construed to limit, the present invention.

### EXAMPLE I

In an 1L round-bottom flask, alpha-tocopherol (20 g, 46 mM) was dissolved in anhydrous acetonitrile (300 ml) by stirring under a nitrogen atmosphere. Pyridine (8 ml, 103 mM) was added thereto and stirred for 10 min. And then, cyclopropane carbonyl chloride solution (150 ml) which made through dissolving the cyclopropane carbonyl chloride (29.5 ml, 245 mM) in acetonitrile, was added slowly under a nitrogen atmosphere by using a dropping funnel (the molar ratio of each reactant, alpha-tocopherol:cyclopropane carbonyl chloride:pyridine, is 1.0:5.3:2.2).

After completion of the addition for about 1 hour, the mixture was stirred at room temperature. The progress of the reaction was monitored by a thin layer chromatography or gas chromatography. As a result of observation as time, it was found that the reaction completely proceeded after 4 hours since the stirring. Henceforth, the acetonitrile solvent was removed from the reaction mixture by use of rotary evaporator.

The residue was dissolved in diethylether (200 ml) and the solution was moved to a separate funnel. And then, the organic layer was washed with distillation water (200 ml * 1), followed by IN aqueous hydrochloride (150 ml * 2), and followed by distillation water (150 ml * 2) again. The resulting organic layer was dried over magnesium sulfate (MgSO₄) and filtered, and the solvent was removed from the organic layer by use of rotary evaporator.

As a result of HPLC, alpha-tocopherol cyclopropylate having 95% purity was obtained, and isolated and purified through tube chromatography on silica gel eluting with hexane/ethyl acetate mixture (10:1, v/v) to afford pure alpha-tocopherol cyclopropylate as a pale yellow oil (20.1 g): Yield 87 %.

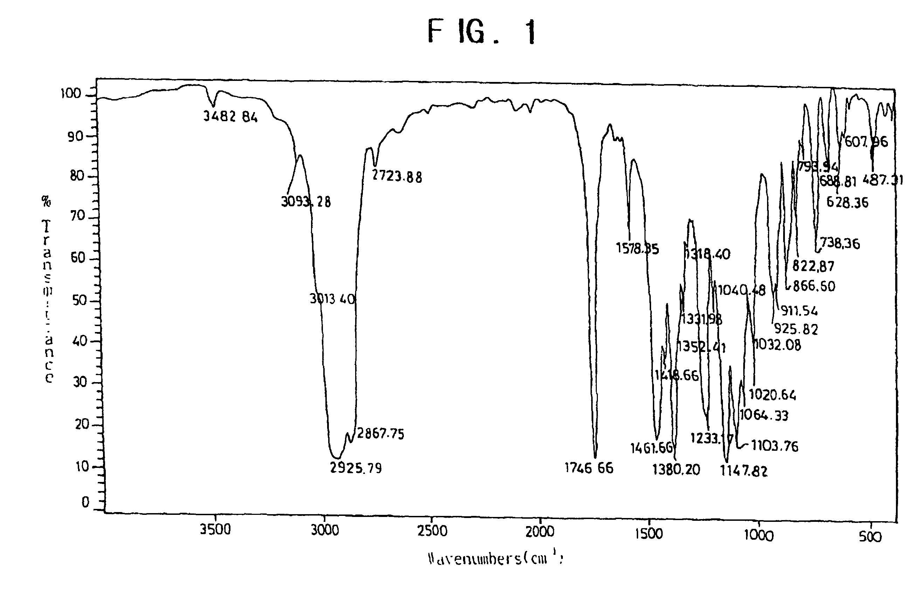

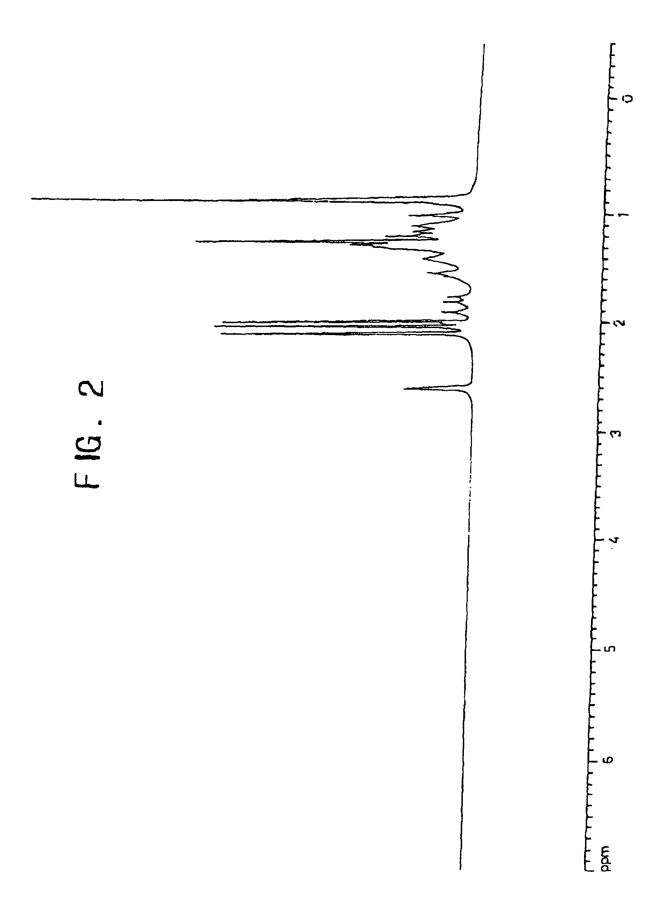

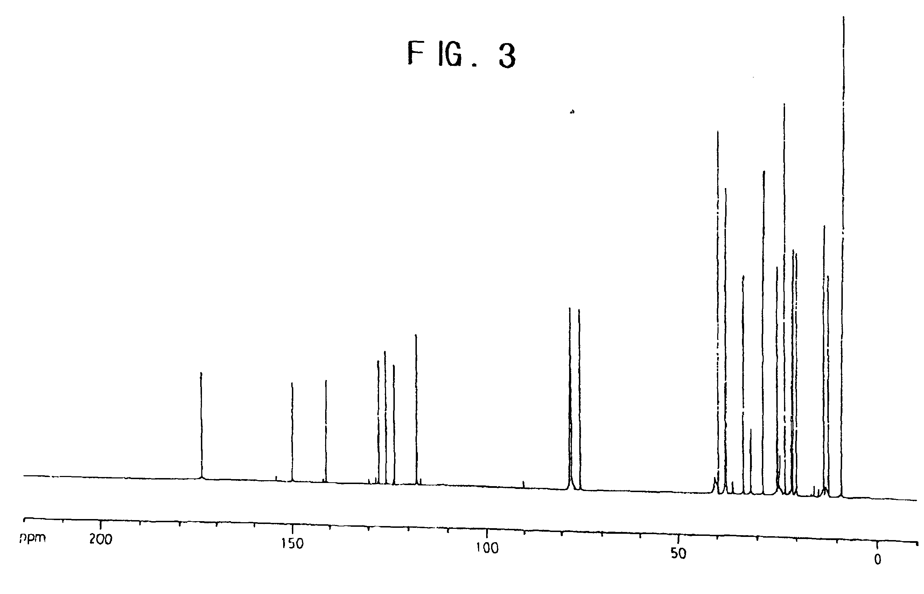

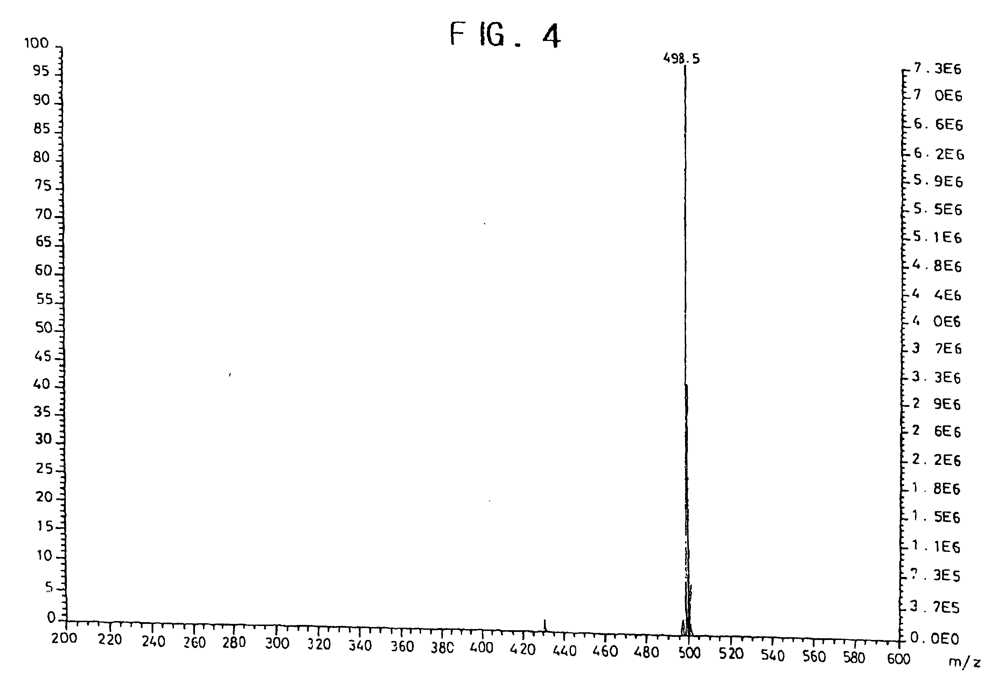

Its structure was identified by infrared spectrometry (Fig. 1), proton nuclear magnetic resonance spectroscopy (¹H-NMR, Fig. 2), carbon nuclear magnetic resonance spectroscopy (Fig. 3) and mass spectroscopy (Fig. 4).

### EXAMPLE II

The reaction of alpha-tocopherol with cyclopropane carbonyl chloride was carried out in the presence of pyridine, in the same manner with Example I, except that the amount of alpha-tocopherol, cyclopropane carbonyl chloride and pyridine was 11.6 mM, 23.1 mM and 20.0 mM (the ratio of moles = 1.0:2.0:1.7), respectively. This reaction was performed in the same method as that of Example I, to produce pure alpha-tocopherol cyclopropylate (3.47 g, yield 60%).

### EXAMPLE III

Alpha-tocopherol cyclopropylate was prepared in the same manner with Example I, except for using dichloromethane, instead of acetonitrile, and that the alpha-tocopherol, the cyclopropane carbonyl chloride and the pyridine are present at a molar ratio of 1.0:5.3:2.2. The reaction was performed at room temperature for 4 hours.

Gas chromatography showed that 76% of the alpha-tocopherol used upon reacting was not reacted. The remaining processes of Example I were repeated to produce alpha-tocopherol cyclopropylate (yield 20%).

### EXAMPLE IV

The procedure of Example III was repeated, except for using toluene as solvent. The remaining processes of Example I were repeated to produce alpha-tocopherol cyclopropylate (yield 50%).

### EXAMPLE V

The procedure of Example III was repeated, except for using tetrahydrofuran as solvent. The remaining processes of Example I were repeated to produce alpha-tocopherol cyclopropylate (yield 29%).

### EXAMPLE VI

The procedure of Example III was repeated, except for using benzene as solvent. The remaining processes of Example I were repeated to produce alpha-tocopherol cyclopropylate (yield 5%).

### EXAMPLE VII

The procedure of Example III was repeated, except for using toluene as solvent. The remaining processes of Example I were repeated to produce alpha-tocopherol cyclopropylate (yield 5.5%).

### EXAMPLE VIII

The reaction was carried out in the presence of acetonitrile, in the same manner with Example I, except that the alpha-tocopherol, the cyclopropane carbonyl chloride and the pyridine are present at a molar ratio of 1.0:1.9:2.1. The reaction was performed at room temperature for 4 hours.

Gas chromatography showed that 30% of the alpha-tocopherol used upon reacting was not reacted. The remaining processes of Example I were repeated to produce alpha-tocopherol cyclopropylate (yield 66%).

### EXAMPLE IX

The reaction was carried out in the presence of acetonitrile, in the same manner with Example T, except that the alpha-tocopherol, the cyclopropane carbonyl chloride and the pyridine are present at a molar ratio of 1.0:3.8:2.1. The reaction was performed at room temperature for 4 hours.

Gas chromatography showed that 12% of the alpha-tocopherol used upon reacting was not reacted. The remaining processes of Example I were repeated to produce alpha-tocopherol cyclopropylate (yield 83%).

### EXAMPLE X

The reaction was carried out in the presence of acetonitrile, in the same manner with Example I, except that the alpha-tocopherol, the cyclopropane carbonyl chloride and the pyridine are present at a molar ratio of 1.0:6.5:2.1. The reaction was performed at room temperature for 4 hours.

Gas chromatography showed that the alpha-tocopherol used upon reacting was completely converted. The remaining processes of Example I were repeated to produce alpha-tocopherol cyclopropylate (yield 95%).

### INDUSTRIAL APPLICABILITY

As described hereinbefore, the compounds according to the present invention are a kind of novel vitamin E esters which are prepared by reacting the alpha-tocopherol and cyclopropane carboxylic acid. The alpha-tocopherol, a fat-soluble essential vitamin E is widely used as an antioxidant, nutrient, medicines, parturifacient and so forth, and the cyclopropane carboxylic acid is recently used as functional group having specific activity in living body, and widely used in commercialized pyrenoids insecticide and antibiotics. The compounds according to the present invention have dual function of vitamin E and cyclopropane carboxylic acid and a good stability against an external factors, especially oxidation. Consequently, the compounds of the invention are fat-soluble vitamin E precursors, as mentioned above, novel vitamin E esters to which cyclopropane carboxylic acid is bound, so that one can expect novel biological activity in itself.

Many modifications and variations of the present invention are possible in light of the above teachings. Therefore, it is to be understood that within the scope of the appended claims, the invention may be practiced otherwise than as specifically described.

## Claims

1. An alpha-tocopherol cyclopropylates, represented by the following formula I: 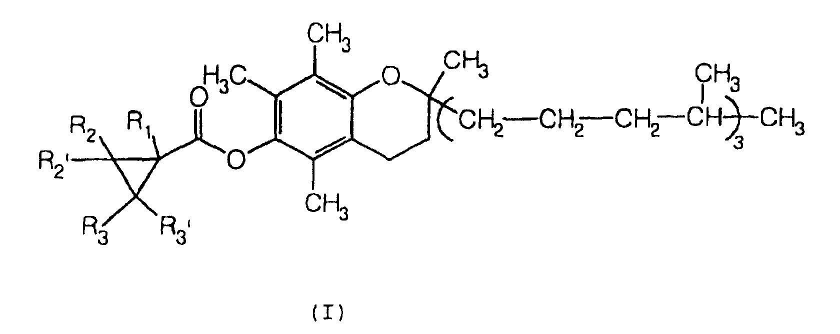 wherein, R₁, R₂, R_{2'}, R₃ and R_{3'}, which may be the same or different, each is a hydrogen atom, a linear or branched C₁-C₁₀ alkyl chain, phenyl, acetyl or halogen.

2. The alpha-tocopherol cyclopropylates in accordance with claim 1, wherein R₁, R₂, R_{2'}, R₃ and R_{3'} each is a hydrogen atom.

3. The alpha-tocopherol cyclopropylates in accordance with claim 1, wherein R₁ is a hycrogen atom and R₂, R_{2'}, R₃ and R_{3'}, which may be the same or different, each is a hydrogen atom, a linear or branched C₁-C₁₀ alkyl chain, phenyl, acetyl or halogen.

4. A method for producing an alpha-tocopherol cyclopropylates represented by the following formula I, comprising reacting cyclopropane carboxylic acid halide produced as shown in the following reaction scheme I by using cyclopropane carboxylic acid represented by the following formula II as starting material with an alpha-tocopherol represented by the following formula III in a solvent in the presence of a base: 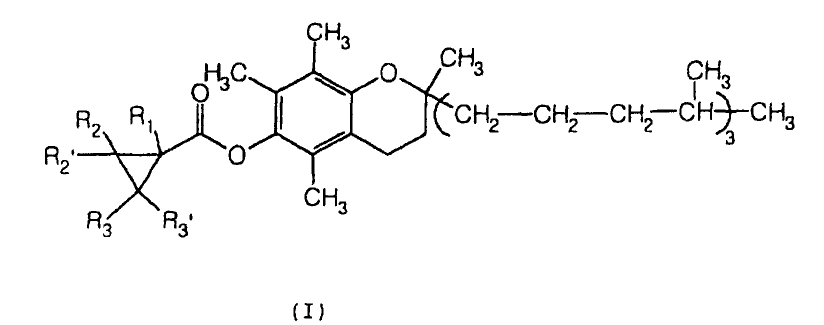 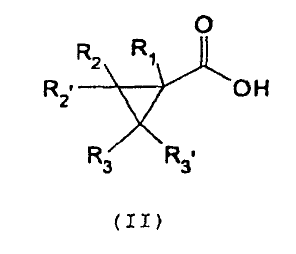 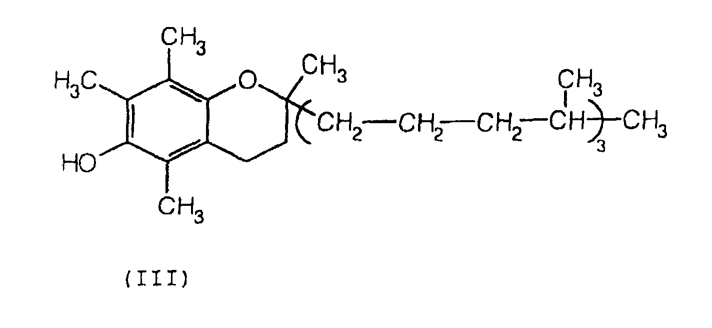 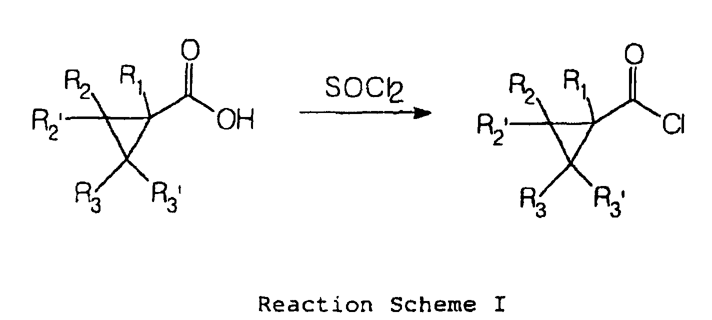 wherein R₁, R₂, R_{2'}, R₃ and R_{3'}, which may be the same or different, each is a hydrogen atom or a linear or branched C₁-C₁₀ alkyl chain, phenyl, acetyl or halogen.

5. The method in accordance with claim 4, wherein said solvent is selected from the group consisting of acetonitrile, dichloromethane, tetrahydrofuran, benzene and toluene.

6. The method in accordance with claim 4, wherein said base is selected from the group consisting of pyridine, pyridine/4-(dimethylamino)pyridine and triethylamine.

7. The method in accordance with claim 4, wherein the alpha-tocopherol, the cyclopropane carboxylic acid halide and the base are present at a molar ratio of 1.0 : 1.5-8.0 : 1.2-5.0.

8. THe method in accordance with claim 7, the alpha-tocopherol, the cyclopropane carboxylic acid halide and the base are present at a molar ratio of 1.0 : 4.0-7.0 : 1.5-2.5.

9. The method in accordance with claim 4, wherein the reaction is carried out at a temperature of 0 to 120°C.

10. THe method in accordance with claim 9, wherein the reaction is carried out at a temperature range from room temperature to 35°C.

11. The method in accordance with claim 4, wherein the reaction is carried out for 4 to 5 hours.

## Patentansprüche

1. α-Tocopherolcyclopropylate, dargestellt durch die folgende Formel I: 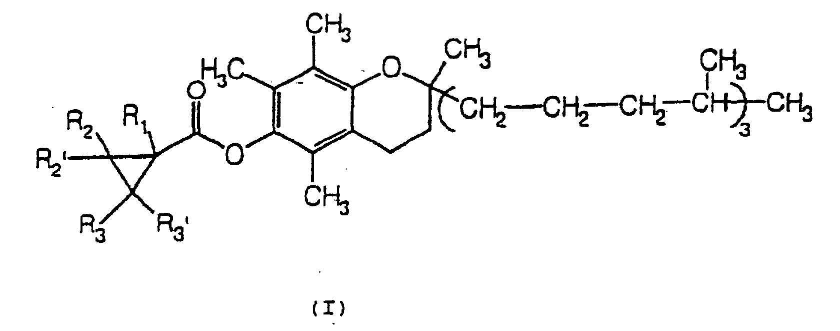 wobei R₁, R₂, R_{2'}, R₃ und R_{3'}, die gleich oder unterschiedlich sein können, jeweils ein Wasserstoffatom, eine lineare oder verzweigte C₁-C₁₀-Alkylkette, Phenyl, Acetyl oder Halogen sind.

2. α-Tocopherolcyclopropylate nach Anspruch 1, wobei R₁, R₂, R_{2'}, R₃ und R_{3'} jeweils ein Wasserstoffatom sind.

3. α-Tocopherolcyclopropylate nach Anspruch 1, wobei R₁ ein Wasserstoffatom ist und R₂, R_{2'}, R₃ und R_{3'}, die gleich oder unterschiedlich sein können, jeweils ein Wasserstoffatom, eine lineare oder verzweigte C₁-C₁₀-Alkylkette, Phenyl, Acetyl oder Halogen sind.

4. Verfahren zur Herstellung von α-Tocopherolcyclopropylaten, dargestellt durch die Formel I, umfassend das Umsetzen eines Cyclopropancarbonsäurehalogenids, das wie in dem folgenden Reaktionsschema I gezeigt unter Verwendung von Cyclopropancarbonsäure, dargestellt durch die folgende Formel II, als Ausgangsmaterial hergestellt wird, mit einem α-Tocopherol, dargestellt durch die folgende Formel III, in einem Lösungsmittel in Gegenwart einer Base: 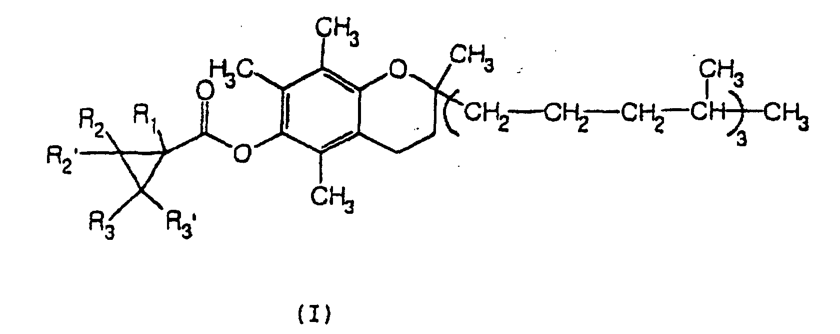 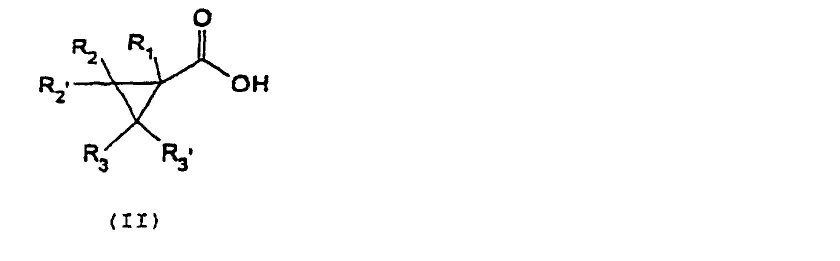 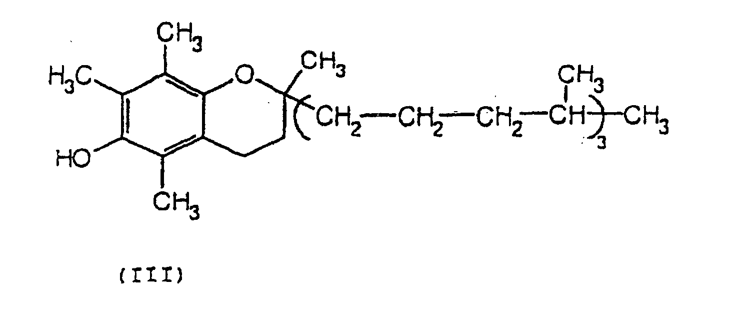 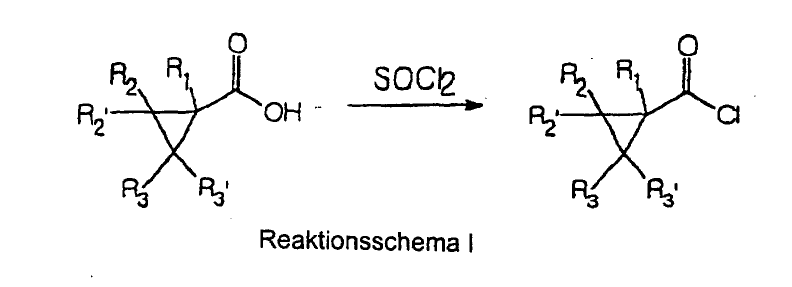 wobei R₁, R₂, R_{2'}, R₃ und R_{3'}, die gleich oder unterschiedlich sein können, jeweils ein Wasserstoffatom oder eine lineare oder verzweigte C₁-C₁₀-Alkylkette, Phenyl, Acetyl oder Halogen sind.

5. Verfahren nach Anspruch 4, wobei das Lösungsmittel aus der Gruppe, bestehend aus Acetonitril, Dichlormethan, Tetrahydrofuran, Benzol und Toluol, ausgewählt ist.

6. Verfahren nach Anspruch 4, wobei die Base aus der Gruppe, bestehend aus Pyridin, Pyridin/4-(Dimethylamino)pyridin und Triethylamin, ausgewählt ist.

7. Verfahren nach Anspruch 4, wobei das α-Tocopherol, das Cyclopropancarbonsäurehalogenid und die Base in einem Molverhältnis von 1,0 : 1,5 bis 8,0 : 1,2 bis 5,0 vorhanden sind.

8. Verfahren nach Anspruch 7, wobei das α-Tocopherol, das Cyclopropancarbonsäurehalogenid und die Base in einem Molverhältnis von 1,0 : 4,0 bis 7,0 : 1,5 bis 2,5 vorhanden sind.

9. Verfahren nach Anspruch 4, wobei die Umsetzung bei einer Temperatur von 0 bis 120°C durchgeführt wird.

10. Verfahren nach Anspruch 9, wobei die Umsetzung in einem Temperaturbereich von Raumtemperatur bis 35°C durchgeführt wird.

11. Verfahren nach Anspruch 4, wobei die Umsetzung für 4 bis 5 Stunden durchgeführt wird.

## Revendications

1. Cyclopropylate d'alpha-tocophérol, représenté par la formule I suivante : 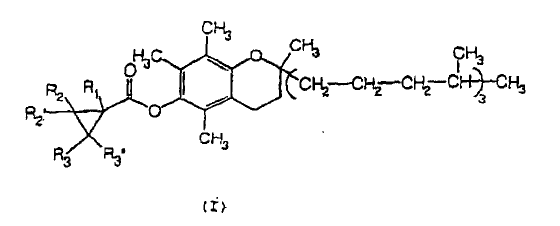 dans laquelle R₁, R₂, R_{2'}, R₃ et R_{3'}, qui peuvent être identiques ou différents, sont chacun un atome d'hydrogène, une chaîne alkyle en C₁-C₁₀ linéaire ou ramifiée, un phényle un acétyle ou un halogène.

2. Cyclopropylate d'alpha-tocophérol selon la revendication 1, dans lequel R₁, R₂, R_{2'}, R₃ et R_{3'} sont chacun un atome d'hydrogène.

3. Cyclopropylate d'alpha-tocophérol selon la revendication 1, dans lequel R₁ est un atome d'hydrogène et dans lequel R₂, R_{2'}, R₃ et R_{3'}, qui peuvent être identiques ou différents, sont chacun un atome d'hydrogène, une chaîne alkyle en C₁-C₁₀ linéaire ou ramifiée, un phényle, un acétyle ou un halogène.

4. Procédé pour produire un cyclopropylate d'alpha-tocophérol représenté par la formule I suivante, comprenant la réaction d'un halogénure d'acide carboxylique de cyclopropane comme cela est illustré sur le schéma réactionnel I suivant en utilisant un acide carboxylique de cyclopropane représenté par la formule II suivante comme matériau de départ avec un alpha-tocophérol représenté par la formule III suivante dans un solvant en présence d'une base : 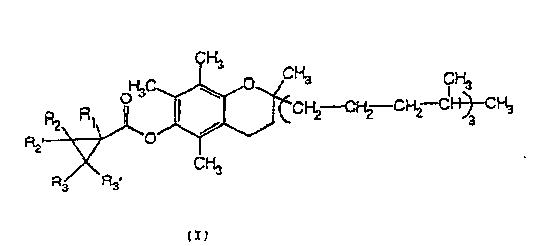 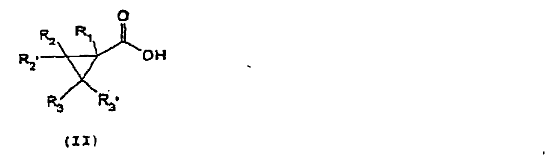 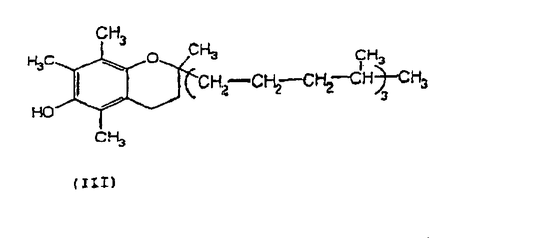 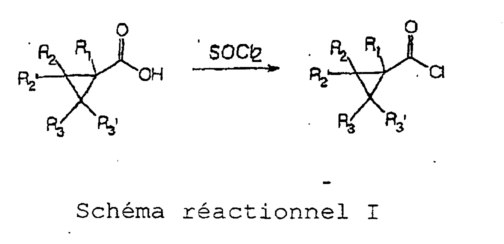 dans lesquels R₁, R₂, R_{2'}, R₃ et R_{3'}, qui peuvent être identiques ou différents, sont chacun un atome d'hydrogène ou une chaîne alkyle en C₁-C₁₀ linéaire ou ramifiée, un phényle, un acétyle ou un halogène.

5. Procédé selon la revendication 4, dans lequel ledit solvant est choisi parmi le groupe consistant en l'acétonitrile, le dichlorométhane, le tétrahydrofuranne, le benzène et le toluène.

6. Procédé selon la revendication 4, dans lequel ladite base est choisie parmi le groupe consistant en la pyridine, la pyridine/4-(diméthylamino)pyridine et la triéthylamine.

7. Procédé selon la revendication 4, dans lequel l'alpha-tocophérol, l'halogénure d'acide carboxylique de cyclopropane et la base sont présents à un rapport molaire de 1:1,5-8:1,2-5.

8. Procédé selon la revendication 7, dans lequel l'alpha-tocophérol, l'halogénure d'acide carboxylique de cyclopropane et la base sont présents à un rapport molaire de 1:4-7:1,5-2,5.

9. Procédé selon la revendication 4, dans lequel la réaction est effectuée à une température de 0 à 120°C.

10. Procédé selon la revendication 9, dans lequel la réaction est effectuée dans une plage de températures allant de la température ambiante à 35°C.

11. Procédé selon la revendication 4, dans lequel la réaction est effectuée pendant 4 à 5 heures.
